# EUROPÄISCHE PATENTSCHRIFT

(11) **EP 1 601 285 B1**
(45) Veröffentlichungstag und Bekanntmachung des Hinweises auf die Patenterteilung: **14.10.2009**
(21) Anmeldenummer: 04718910.5
(22) Anmeldetag: 10.03.2004
(51) Int. Cl.: A61B 5/00, G01N 21/47

(54) **BLUTOPTODE**
BLOOD OPTODE
OPTODE DESTINEE AU SANG

(30) Priorität: 13.03.2003 DE 10311408
(43) Veröffentlichungstag der Anmeldung: 07.12.2005
(73) Patentinhaber: Nirlus Engineering AG, 23560 Lübeck (DE)
(72) Erfinder: HERRMANN Vera, 23560 Luebeck (DE)
(74) Vertreter: von dem Borne, Andreas
(86) Internationale Anmeldenummer: PCT/DE2004/000470
(87) Internationale Veröffentlichungsnummer: WO 2004/080295

(56) Entgegenhaltungen:
- EP-A- 0 832 599
- WO-A-02/08740
- WO-A-02/059580

## Beschreibung

Die Erfindung betrifft ein medizintechnisches Verfahren und eine Vorrichtung, um nichtinvasiv die Konzentration von Blutbestandteilen, insbesondere die Hämoglobinkonzentration oder die Sauerstoffsättigung des Blutes, in großen Blutgefäßen zu messen.

Lebendes Gewebe ist für elektromagnetische Strahlung im roten und im Infrarot-Bereich (Wellenlänge 550 nm < λ < 1000 nm) weitgehend transparent. Dieses sog. "biologische Fenster" wird zu längeren Wellenlängen hin durch starke Absorptionsbanden des Wassers und zu kürzeren durch solche des Hämoglobins begrenzt. Prinzipiell ist es in diesem Bereich möglich, in das Gewebe in Tiefen von einigen mm bis zu einigen cm "hineinzusehen".

Im letzten Jahrzehnt hat sich insbesondere die sog. Pulsoximetrie zu einem der wichtigsten Monitoringverfahren zur Beobachtung des Patienten auf der Intensivstation und im Operationssaal entwickelt. Dabei wird die sog. Sauerstoffsättigung des Blutes gemessen, d. h. das Verhältnis der Konzentration des mit Sauerstoff beladenen Hämoglobins zum gesamten Hämoglobin. Dies geschieht unter Messung der Absorption im Durchlicht oder der Remission im rückgestreuten Licht an gut durchblutetem Gewebe (z. B. Fingerkuppe oder Ohrläppchen) bei zwei verschiedenen Wellenlängen. Die benutzten Wellenlängen liegen üblicherweise um λ = 660 nm (wo das von Sauerstoff freie Hämoglobin viel stärker absorbiert als das mit Sauerstoff beladene) und um λ = 940 nm (wo die Verhältnisse umgekehrt sind). Benutzt wird bei der Messung die vom Herzschlag erzeugte Modulation des Absorptionssignals. Das Wechselsignal wird dem interessierenden arteriellen Anteil zugerechnet, dem als Untergrund vorhandenen Gleichsignal die Absorption durch venöses Blut und Gewebe. Die Bestimmung der Sauerstoffsättigung als relative Größe ist dabei trotz einiger prinzipieller Schwierigkeiten in praxi mit für den klinischen Einsatz ausreichender Genauigkeit möglich.

Die medizinische Fachliteratur belegt aber die dringliche Notwendigkeit einer bettseitigen, d.h. kontinuierlichen, nichtinvasiven Bestimmung der Sauerstoffsättigung des Hämoglobins in großen Blutgefäßen. Die Blutuntersuchung in großen, sog. "zentralen", d.h. herznahen Gefäßen ist mit bisher üblichen Verfahren zur Bestimmung der Sauerstoffsättigung wegen der sog. "Zentralisierung" (d. i. Mangeldurchblutung der Peripherie) von Notfallpatienten nicht möglich. Primär besteht daher Bedarf an einem Verfahren, das arteriell anwendbar ist, z.B. auf die arteria carotis interna, wenn möglich auch auf große Venen, z. B. die vena iugularis interna, weil die Differenz in der Sauerstoffsättigung wesentliche Aufschlüsse über die Sauerstoffversorgung des Gehirns gibt.

Die Schwierigkeit einer Messung an großen Blutgefäßen ist, dass deren zentrale Lage im Körper eine Transmissionsmessung unmöglich macht, während Remission vorwiegend durch diffuse Rückstreuung von Photonen erfolgt. Man unterscheidet dabei zwischen ballistischen oder quasi-ballistischen Photonen, die keine oder geringe Wechselwirkung mit dem Gewebe erfahren und dieses daher als erste wieder verlassen, und diffusen Photonen, deren Weg durch das Gewebe durch zahlreiche Streuprozesse gekennzeichnet ist. Ballistische Photonen sind für die Blutanalyse von untergeordneter Bedeutung. Für menschliches Gewebe ist der Streukoeffizient µ*ₛ* sehr viel größer als der Absorptionskoeffizient µ*ₐ*. µ*ₛ* liegt typisch in der Größenordnung von 10 cm⁻¹, so dass für Schichtdicken ≥ 1 - 2 mm kein Lichtfokus im streuenden Medium mehr erzeugt werden kann. Die Beleuchtung tiefer liegender Schichten erfolgt daher quasi-isotrop. Die Zuordnung rückgestreuten Lichts zu einem bestimmten Ort der Streuung ist das wesentliche Problem.

In der Druckschrift DE 196 40 807 A1 wird eine Vorrichtungen zur Detektion diffuser Photonen vorgeschlagen, die in Rückstreurichtung, d.h. in Nachbarschaft der Lichtquelle(n), messen und zur Bestimmung der Sauerstoffkonzentration in Blut und Gewebe dienen soll. Dabei wird ein empirisch bekannter Zusammenhang zwischen dem Abstand des Austrittspunktes rückgestreuter Photonen vom Eintrittspunkt (Lichtquelle, Faserende) und der mittleren Eindringtiefe dieser Photonen auf ihrem Weg durch das Gewebe benutzt, um durch die Wahl dieses Abstandes die Beobachtungstiefe zu kontrollieren.

Die Druckschrift DE 196 34 152 A1 verwendet eine sehr ähnliche Messanordnung und benutzt, dass Anteile des eingestrahlten, kohärenten Lichts durch elastische und unelastische Streuprozesse phasen- bzw. frequenzverschoben und mit den ungestörten Anteilen überlagert werden, was auf ein Speckle-Muster führt. Die ortsaufgelöste Messung des Speckle-Musters ermöglicht eine Analyse des austretenden Streulichts hinsichtlich seines Leistungsspektrums im Vergleich zu dem des eingestrahlten Lichts. Beispielsweise lassen sich anhand von wiederholten Frequenzverschiebungen durch unelastische Streuung an Blut Aussagen treffen über die mittlere Zahl der Streuprozesse pro Photon auf dem Weg des Lichts durch das Gewebe. Die Anwendung einer Filterprozedur erlaubt dann das Diskriminieren von Photonen, die eine vorgegebene Mindest- oder Höchstzahl an Streuprozessen hinter sich haben und somit eine relativ stark eingegrenzte Eindringtiefe besitzen.

In der Druckschrift WO 02/08740 A2 wird der fortgeschrittenste Stand der Technik für eine Messvorrichtung mit rückgestreuten Photonen vorgestellt. Ausgehend von der bekannten Wechselwirkung des Lichts mit einem im Gewebe vorhandenen Ultraschallfeld, wird aus der so hervorgerufenen Beeinflussung der Phasen der elektromagnetischen Wellen auf die genaue Position der verantwortlichen Streuzentren in einem dreidimensionalen Messgebiet geschlossen. Ein für diesen Zweck geeignetes Ultraschallfeld wird durch entweder eine einzelne bewegliche oder durch ein ganzes Array von Schallquellen in Kontakt mit dem Gewebe erzeugt. Wesentlich für die erforderliche, komplexe Struktur des Ultraschallfeldes ist die genaue Steuerung der Quellen hinsichtlich der Einhaltung von Phasenverzögerungen und Repetitionszeiten und/oder von Frequenzunterschieden. Ähnlich kompliziert gestaltet sich die umfangreiche Datenanalyse auf der Detektorseite. Das Gerät ist zur 3D-Bild-gebenden Tomographie bei der Frage nach der Blutversorgung von Gewebe, etwa im Vorfeld der Tumorbekämpfung, vorgesehen.

Allen genannten Verfahren ist gemein, dass der Beitrag zum Messsignal von Blut aus dem Innern eines großen Blutgefäßes nicht isoliert betrachtet werden kann, bzw. im Fall der WO 02/08740 A2 nur mit größerem Aufwand zu ermitteln ist. Für das kontinuierliche Monitoring von Notfallpatienten ist ein einfaches, robustes, schnelles und kostengünstiges System erforderlich. Es ist Aufgabe der Erfindung, ein solches bereitzustellen.

Die Aufgabe wird gelöst durch die Merkmale des Anspruchs 1. Die Unteransprüche geben vorteilhafte Ausgestaltungen der Erfindung an.

Das erfindungsgemäße Verfahren greift den Grundgedanken der WO 02/08740 A2 auf, verzichtet dabei aber auf jegliche Frequenz- oder Phasenanalyse des verwendeten Lichts. Vielmehr werden ausschließlich Lichtintensitäten betrachtet, d.h. Photonen gezählt, was auf der Messseite eine sehr einfache Detektoranordnung und Auswertung erlaubt. Zudem ist es nicht erforderlich, kohärentes Licht zu verwenden, wenngleich auch hier bevorzugt gängige Laserdioden eingesetzt werden. Folgende Abbildungen dienen der Erläuterung der Erfindung:
Figur 1 zeigt eine schematische Darstellung des Verfahrens und einer Vorrichtung zur Durchführung des Verfahrens.
Figur 2 beschreibt den Effekt, der die präzise Lokalisierung der zum Messsignal beitragenden Streuzentren erlaubt.
Figur 3 zeigt den gemessenen Verlauf des Druckfeldes einer fokussierten Ultraschallquelle im Laborexperiment.

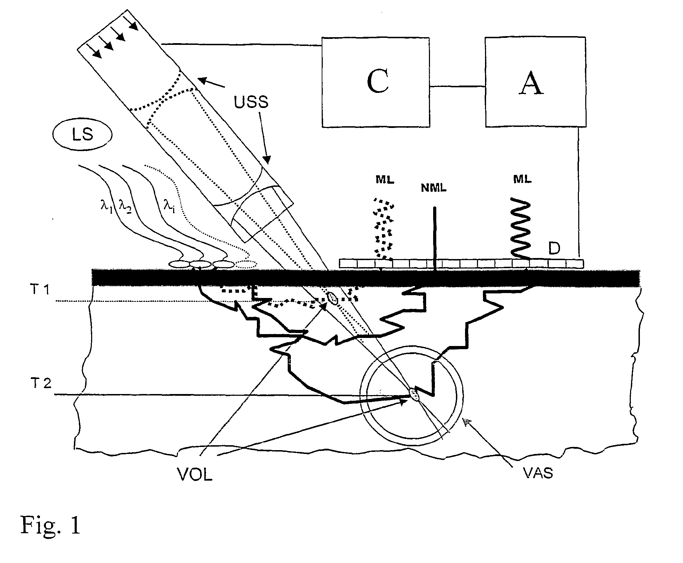

In Figur 1 sendet zunächst eine Lichtquelle (LS) Licht, bevorzugt monochromatisches Licht mit einer oder mehreren diskreten Wellenlängen, z.B. Laserlicht, in das Gewebe. Die Lichtwellenlängen λₗ bis λᵢ sind dabei so gewählt, dass bevorzugt Streuung an selektierten Blutbestandteilen, insbesondere an sauerstoffreichem und sauerstoffarmem Hämoglobin, stattfindet. Ein signifikanter Anteil des eingestrahlten Lichts tritt nach zahlreichen Streuprozessen an einer Vielzahl von Austrittsstellen wieder aus. Ein Matrixdetektor (D), der aus flächig nebeneinander angeordneten, lichtempfindlichen Pixeln besteht, die ein zur Lichtintensität proportionales, elektrisches Signal erzeugen, ist auf der Hautoberfläche so angeordnet, dass die dem Einstrahlpunkt benachbarten Austrittsstellen vom Detektor bedeckt sind. Die Abmessungen des Detektors müssen mit der angestrebten Eindringtiefe des Lichts korrespondieren (s. o.), also mit der Tiefe des zu untersuchenden Blutgefäßes. Eine mit dem Detektor verbundene Auswerteeinheit (A) summiert die Signale des Detektors und misst die rückgestreute, aus dem Gewebe austretende Lichtintensität integrierend über alle Pixel und über ein fest gewähltes Zeitfenster. Bei gleich bleibender Beleuchtung ist diese Lichtintensität zeitlich konstant.

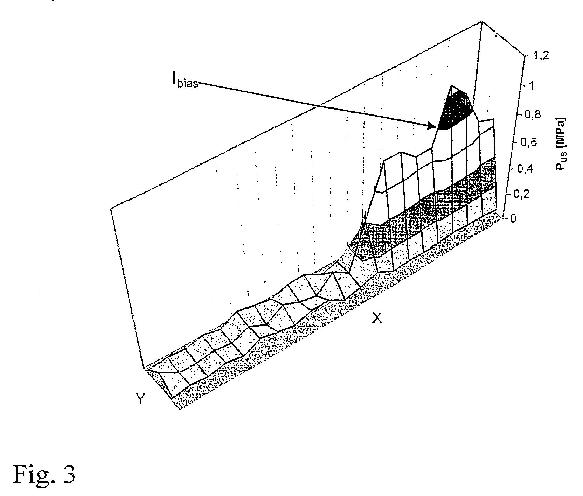

Eine Ultraschallquelle (USS) mit verstellbarem Fokus emittiert nun zusätzlich gepulste Ultraschallstrahlung bekannter Frequenz f_{US} in das Medium. Das Ultraschallwellenfeld besitzt durch die Fokussierung einen von der Schallquelle beabstandeten Fokus, in dem die Amplitude des Wellenfeldes maximal ist. Figur 3 zeigt als Beispiel das experimentell gemessene Druckfeld einer USS in einem Reagenzglas (Durchmesser 16 mm, Y-Achse). Die X-Achse überstreicht einen Abstandsbereich von 44 mm, der Druck P_{US} nimmt im Fokus einen Maximalwert knapp über 1 MPa an, was dem medizinisch zulässigen Maximalwert entspricht.

Die Vorrichtung zur Einstrahlung des Ultraschalls umfasst wenigstens eine Vorrichtung zur Erregung der Wellen (USS) und eine elektronische Steuereinheit (C). Insbesondere kontrolliert die Steuereinheit die Ultraschallfrequenz, die Pulsfolge und die Position des Fokus. Pulsdauer und Repetitionszeit der Ultraschallstrahlung sind sehr viel kleiner gewählt als das Zeitfenster der Intensitätsmessung des rückgestreuten Lichts. Die Lichtmessung ist mit dem Beginn jedes US-Pulses getriggert und zeichnet die austretende Lichtintensität über die Dauer eines Pulses auf. Durch Kumulation des Signals über eine Vielzahl von Pulsen wird in wenigen Sekunden eine statistische Mittelung erreicht.

Das Ultraschallwellenfeld verursacht durch Wechselwirkung mit Blut und Gewebe (lokale Dichtevariationen) Änderungen der optischen Eigenschaften, insbesondere des Reflexions- bzw. Streuvermögens. Als Folge davon enthält die gemittelte, rückgestreute Lichtintensitätals Verteilung über die Zeitspanne eines einzelnen Pulses - einen Anteil, der mit der Frequenz f_{US} moduliert ist. Die Auswerteeinheit eliminiert am Ende des Zeitfensters der Messung zeitlich konstante Anteile des Signals, führt eine Fouriertransformation von Zeit- zu Frequenzkoordinaten durch und isoliert die Fourierkomponente, die mit f_{US} korrespondiert. Diese Fourierkomponente dominiert typischerweise das Frequenzspektrum und ihr Betrag ist im Weiteren die einzig verbleibende Messgröße. Sie wird kurz als M bezeichnet.

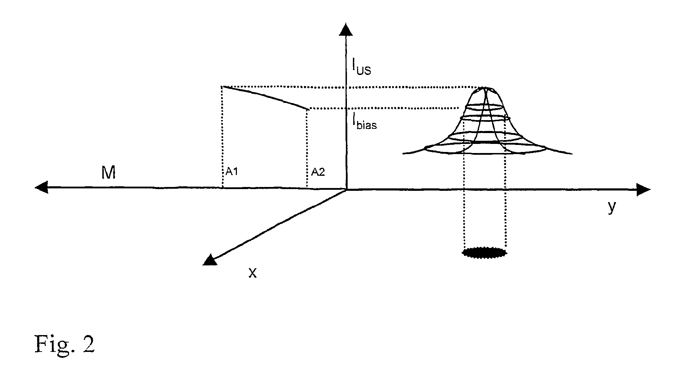

M hängt sowohl vom physikalischen Streuvermögen des Blutes für die benutzten Lichtwellenlängen als auch von der Amplitude des Ultraschallwellenfeldes I_{US} ab. Man findet experimentell eine annähernd lineare Abhängigkeit M(I_{US}), sofern I_{US} - im Fokus - einen Schwellwert I_{bias} überschreitet. Andernfalls ist M nicht mehr messbar. Figur 2 stellt diesen Sachverhalt schematisch dar: Der räumliche Verlauf der Wellenfeldamplitude wird dort durch eine gaußartige Verteilung bezüglich der Koordinaten x und y angedeutet. M variiert zwischen A1 und A2. Der Wert von I_{bias} liegt für eine gegebene Konfiguration des Messaufbaus (u. a. Schallquelle, Lichtquelle, Medium, Auflösung des Detektors etc.) fest und definiert das Volumen eines Raumgebiets im Ultraschallfokus (vgl. auch Figur 3). I_{US} liegt nur innerhalb dieses Gebiets oberhalb von I_{bias}. Das Signal M rührt alleine von Streuprozessen aus diesem Gebiet her.

In Figur 1 sind drei exemplarische Lichtwege mit Vielfachstreuung dargestellt. Zwei der Wege durchlaufen dabei den - verschiebbaren - Fokus und führen auf modulierte Lichtintensitäten (ML) am Detektor (D). Einer hingegen durchläuft keinen Fokus, so dass nicht moduliertes Licht (NML) am Detektor eintrifft, das von der Auswerteeinheit herausgefiltert wird.

Die Position des Ultraschallfokus wird von außen vorgegeben, ist also bekannt. Das zum Signal beitragende Gebiet durchmisst i. a. wenige Millimeter und kann durch Steuerung der Schallquelle im Gewebe verschoben werden. Insbesondere kann der Fokus vollständig im Innern eines herznahen, großen Blutgefäßes positioniert werden. Eine solche Platzierung des Fokus ist mit Hilfe des Doppler-Effekts zu erzielen. Bekanntlich führt die Wechselwirkung von Ultraschall mit einem relativ zur Schallquelle bewegten Medium - hier: fließendes Blutzu einer Frequenzverschiebung fus → f_{US}^{(D)} der rückgestreuten bzw. reflektierten Welle. Dies kann bereits von gängigen, für medizinische Zwecke vorgesehenen Ultraschallquellen mit Fokuskontrolle durch Hautmessung registriert und zum Auffinden von Blutgefäßen (VAS in Figur 1) ausgenutzt werden.

Bei der oben beschriebenen Fouriertransformation der am Detektor registrierten zeitlichen Verteilung der Lichtintensität hat der vollständige Übergang des Fokus in ein großes Blutgefäß zur Folge, dass die dominante Fourierkomponente bei f_{US}^{(D)} anstelle von f_{US} auftritt. Bei gleich bleibendem Streuvermögen des Mediums, d.h. etwa bei Übergang des Fokus von stehendem in fließendes Blut ohne Änderung weiterer Parameter, bleibt das Messsignal M unverändert. Die Verschiebung seiner spektralen Lage lässt aber nun den Rückschluss auf die Fließgeschwindigkeit des Mediums (hier: Blutes) zu, was zur Berechnung der Sauerstoffkonzentration ebenfalls von der Steuereinheit (C) der Ultraschallquelle an die Auswerteeinheit (A) übermittelt wird.

Da nach der abgeschlossenen Positionierung des Fokus die Tiefe des zu beobachtenden Blutgefäßes unter der Hautoberfläche bekannt ist, benutzt die Auswerteeinheit diese Information, um die am Detektor eintreffende, f_{US}-modulierte Lichtintensität dahingehend zu korrigieren, dass für das Gewebe spezifische Absorptions- und Streuverluste pro durchquerter Wegstrecke kompensiert werden. Vorzugsweise kann hierfür das Wasserabsorptionsmaximum bei λ = 975 nm als innere Referenz betrachtet werden. Besonders bevorzugt zieht man das Absorptionsverhältnis von Indocyangrün (ICG) zu Wasser heran, das sich als unabhängig vom streuenden Medium erweist. Die so korrigierten Messdaten ermöglichen das Schließen auf absolute Werte für die optischen Parameter innerhalb des durch den Fokus definierten Messvolumens. Diese werden für die verschiedenen eingestrahlten Lichtwellenlängen separat bestimmt, um mit bekannten Verfahren auf die Konzentration des zu messenden Blutbestandteils, insbesondere auf das Verhältnis von sauerstoffreichem zu sauerstoffarmem Hämoglobin, zu schließen. Das beschriebene Vorgehen stellt sicher, dass überhaupt nur Blut aus dem interessierenden Blutgefäß untersucht wird.

Von besonderem Vorteil ist, dass der apparativ vorgegebene Wert für I_{bias} weiterhin auf der Auswertungsseite rechnerisch erhöht werden kann. Dadurch lässt sich das betrachtete Volumen im Ultraschallfokus gezielt verkleinern. Die genaue Größe des Volumens ist bestimmbar, und in Kombination mit der ebenfalls gemessenen Fließgeschwindigkeit des Blutes kann die pro Zeiteinheit zum Messsignal beitragende Blutmenge absolut berechnet werden.

Eine bevorzugte Ausgestaltung des Verfahrens besteht in der Untersuchung von Gefäßwandablagerungen (Plaque). Durch systematisches Schieben eines hinreichend klein gewählten Fokusvolumens durch ein großes Gefäß kann festgestellt werden, ob sich der Fokus ganz oder teilweise in Blut, Gewebe oder einem anderen Medium befindet. Ablagerungen an Gefäßwänden gefährden die Durchblutung und können ein lebensbedrohliches Risiko bedeuten. Dabei werden unterschiedlich strukturierten Ablagerungen durchaus verschiedene Risikopotenziale zugeschrieben. Gefährliche heterogene Ablagerungen müssen grundsätzlich operativ entfernt werden, während eher homogene Beläge in gewissem Umfang geduldet werden können. Bis jetzt war eine nichtinvasive Möglichkeit der Unterscheidung aber nicht gegeben. Das hier vorgestellte Verfahren gibt Hinweise auf die Struktur der Beläge, was überflüssige Eingriffe vermeiden hilft.

## Patentansprüche

1. Verfahren zur nichtinvasiven Messung der Konzentration von Blutbestandteilen in zentralen Blutgefäßen, insbesondere der Hämoglobinkonzentration oder der Sauerstoffsättigung des Blutes, durch das Messen von rückgestreutem Licht unter Einwirkung einer Ultraschallstrahlung, **gekennzeichnet durch**
a. Fokussieren der Ultraschallstrahlung auf das Innere eines zentralen Blutgefäßes,
b. Vorgabe einer festen Pulslänge und Repetitionszeit für die Ultraschallstrahlung,
c. Anordnen einer Lichtquelle und einer benachbarten Detektionseinheit zum Erfassen des rückgestreuten Lichts auf der Hautoberfläche über dem Blutgefäß derart, dass der Abstand zwischen Lichtquelle und der Mehrheit der Lichtrezeptoren der Detektionseinheit mit der Tiefe des untersuchten Blutgefäßes korrespondiert,
d. Beleuchten des Zielgewebes mit wenigstens zwei diskreten Lichtwellenlängen,
e. Messen der rückgestreuten Lichtintensität integrierend über die Detektorfläche und eine Vielzahl von Ultraschallpulsen,
f. Erfassen einer mittleren Lichtintensitätsverteilung über die Dauer eines Pulses,
g. Eliminieren zeitlich konstanter Anteile und Fouriertransformation der Verteilung,
h. Rückschließen auf die Fließgeschwindigkeit des Blutes und sein Rückstreuvermögen für jede der wenigstens zwei Lichtwellenlängen unter Berücksichtigung der ermittelten Tiefe des Blutgefäßes,
i. Rückschließen auf die Menge der zum Signal beitragenden Blutkomponenten aus den ermittelten Streuvermögen,
j. Berechnung von Konzentrationen im Blutgefäß unter Berücksichtigung des zum Signal beitragenden Volumens des Ultraschallfokus und der Blutfließgeschwindigkeit.

2. Verfahren nach Anspruch 1, **dadurch gekennzeichnet, dass** das Volumen des zum Messsignal beitragenden Fokus auf der Auswerteseite verringert wird, indem ein höherer Schwellwert I_{bias} für die mindestens im Messvolumen vorliegende Amplitude des Ultraschallwellenfeldes verlangt wird.

3. Verfahren nach einem der vorangehenden Ansprüche, **dadurch gekennzeichnet, dass** die Position des Fokus so verändert wird, dass der Fokus ein zentrales Blutgefäß kontinuierlich oder in mehreren Schritten durchquert, wobei die Messung der Lichtintensität während der Fokusbewegung oder an den verschiedenen Fokuspositionen dahingehend ausgewertet wird, dass ein mit der Position veränderlicher Durchblutungsgrad des Gewebes ermittelt wird.

4. Verfahren nach einem der vorangehenden Ansprüche, **dadurch gekennzeichnet, dass** nach Fouriertransformation der Lichtintensitätsverteilung in einem gewählten Spektralbereich die dem Betrage nach größte Fourierlcomponente und deren spektrale Lage bestimmt werden.

## Claims

1. A method for the non-invasive measurement of the concentration of blood components in central blood vessels, more preferably the haemoglobin concentration or the oxygen saturation of the blood through the measuring of backscattered light under the influence of ultrasonic radiation, **characterized by**
a. focussing of the ultrasonic radiation on to the interior of a central blood vessel,
b. presetting of a fixed pulse length and repetition time for the ultrasonic radiation,
c. arranging of a light source and an adjacent detection unit to record the backscattered light on the skin surface above the blood vessel in such a manner that the distance between light source and the plurality of the light receptors of the detection unit corresponds with the depth of the examined blood vessel,
d. lighting of the target tissue with at least two discrete light wavelengths,
e. measuring of the backscattered light intensity in an integrating manner over the detector area and a multiplicity of ultrasonic pulses,
f. recording a mean light intensity distribution over the duration of a pulse,
g. eliminating components constant over time and Fourier transformation of the distribution,
h. deducing the flow velocity of the blood and its backscattering power for each of the at least two light wavelengths taking into account the determined depth of the blood vessel,
i. drawing conclusions as to the quantity of the blood components contributing to the signal from the scattering power determined,
j. calculation of concentrations in the blood vessel taking into account the volume of the ultrasonic focus and the blood flow velocity contributing to the signal.

2. The method according to Claim 1, **characterized in that** the volume of the focus contributing to the measurement signal is reduced on the evaluation side in that a higher threshold value I_{bias} is demanded for the amplitude of the ultrasonic wave field at least present in the measurement volume.

3. The method according to any one of the preceding claims, **characterized in that** the position of the focus is changed so that the focus passes through a central blood vessel continuously or in a plurality of steps, wherein the measurement of the light intensity during the focus movement or at the various focus points is evaluated with the objective that a blood circulation degree of the tissue that varies with the position is determined.

4. The method according to any one of the preceding claims, **characterized in that** following the Fourier transformation of the light intensity distribution in a selected spectral range the Fourier component which is largest by amount and its spectral position are determined.

## Revendications

1. Procédé de mesure non invasive de la concentration de composants sanguins dans des vaisseaux sanguins centraux, notamment de la concentration d'hémoglobine ou de la saturation d'oxygène dans le sang par la mesure de lumière rétrodiffusée sous action d'un rayonnement ultrasonique,
**caractérisé par**
a. la focalisation du rayonnement ultrasonique sur l'intérieur d'un vaisseau sanguin central,
b. l'allocation d'une longueur d'impulsions et d'un temps de répétition fixe pour le rayonnement ultrasonique,
c. la disposition d'une source lumineuse et d'une unité de détection voisine pour la détection de la lumière rétrodiffusée sur la surface de la peau au-dessus du vaisseau sanguin, de telle sorte que la distance entre la source lumineuse et la majorité des récepteurs de lumière de l'unité de détection correspondent avec la profondeur du vaisseau sanguin analysé,
d. l'éclairage du tissu cible par au moins deux longueurs d'ondes lumineuses discrètes,
e. la mesure de la lumière rétrodiffusée de façon intégrante via la surface du détecteur et une pluralité d'impulsions ultrasoniques,
f. la détection d'une distribution moyenne d'intensité lumineuse sur la durée d'une impulsion,
g. l'élimination de fractions temporairement constantes et la fraction de Fourier de la distribution,
h. la déduction de la vitesse d'écoulement du sang et de sa capacité de rétrodiffusion pour chacune des aux moins deux longueurs d'ondes lumineuses, sous considération de la profondeur déterminée du vaisseau sanguin,
i. la déduction de la quantité des composants sanguins contribuant au signal à partir de la capacité de rétrodiffusion déterminée,
j. le calcul de concentrations dans le vaisseau sanguin, sous considération du volume du foyer d'ultrasons contribuant au signal et de la vitesse d'écoulement du sang.

2. Procédé selon la revendication 1, **caractérisé en ce que** le volume du foyer contribuant au signal de mesure est réduit côté évaluation **en ce qu'**une valeur seuil plus élevée I_{bias} est réclamée pour l'amplitude des ondes ultrasonores présentes au moins dans le volume de mesure.

3. Procédé selon l'une quelconque des revendications précédentes, **caractérisé en ce qu'**on varie la position du foyer de sorte que le foyer traverse en continu ou en plusieurs étapes un vaisseau sanguin central, la mesure de l'intensité lumineuse étant évaluée pendant le déplacement du foyer ou dans les différentes positions du foyer, en ce sens qu'un taux d'irrigation sanguine du tissu changeant avec la position est déterminé.

4. Procédé selon l'une quelconque des revendications précédentes, **caractérisé en ce qu'**après la transformation de Fourier de la distribution de l'intensité lumineuse dans un domaine spectral choisi, la plus grande composante de Fourier en nombre et la position spectrale de cette dernière sont déterminées.
